# EUROPEAN PATENT APPLICATION

(11) **EP 4 621 408 A1**
(43) Date of publication of application: **24.09.2025**
(21) Application number: 25159574.0
(22) Date of filing: 24.02.2025
(51) Int. Cl.: G01N 33/543, G01N 33/84

(54) **METHOD FOR MANUFACTURING A TEST STRIP WITH IMPROVED SPECIFIC GRAVITY MEASUREMENT**

(30) Priority: 19.03.2024 FR 2402737
(71) Applicant: Withings, 92130 Issy-Les-Moulineaux (FR)
(72) Inventor: Campo, David, 92130 Issy-les-Moulineaux (FR); Nagarajan, Sounderya, 92130 Issy-les-Moulineaux (FR)
(74) Representative: Withings IP

(57) **Abstract**

The present disclosure concerns a method to manufacture a test strip for measuring specific gravity of a urine sample, the method comprising successively:
- providing a polyelectrolyte in a first solution, the polyelectrolyte being poly(methyl vinyl ether-alt-maleic anhydride),
- adding to the first solution a base to neutralize the polyelectrolyte and to bring the first solution at a pH between 10 and 11,
- adding to the first solution a buffering agent to bring and maintain the first solution at a pH between 7.0 and 7.5,
- dipping a paper strip into the first solution,
- dipping the paper strip into a second solution comprising a pH sensitive color indicator.

## Description

### FIELD

The present disclosure relates to a method for manufacturing a test strip for measuring specific gravity of a urine sample. The present disclosure also relates to a test strip for determining the specific gravity of a urine sample urine manufactured by such a method.

The test strip is designed to be arranged in a cartridge configured to be inserted in a station for urine analysis. The station may be installed on the surface of a toilet bowl. The cartridge mounted on the station will be referred to as an analysis device. The station comprises a light source and an optical sensor to carry out an optical analysis on the test strip.

### BACKGROUND

Urine may be a source of useful information about the health of the user. Monitoring the urine of a user may provide information, for example about what the user ingests, about body's waste and excess mineral salts.

In particular, measuring the specific gravity of the urine of a user may be useful for assessing a hydration status, a renal function, for monitoring certain diseases as diabetes and kidney disease, for monitoring response to a medical treatment, for assessing urinary tract disorders such as urinary tract infections (UTI), kidney stones or other conditions affecting urine concentration, for monitoring hyponatremia or hypernatremia, for detecting of heart failure, etc.

A urine-specific gravity test compares the density of urine with the density of water and shows the total concentration of all chemical particles in the urine.

Several methods are available to determine the specific gravity of urine.

The most widely used method, and possibly the least accurate, uses a urinometer. The urinometer is a weighted, bulb-shaped instrument having a cylindrical stem containing a scale calibrated in specific gravity readings. The urinometer method is cumbersome and suffers from the disadvantages of requiring large volumes of urine test sample, from difficult and inaccurate reading of the urinometer scale and from unreliable assays because the urinometer is not regularly recalibrated.

Refractometry provides an indirect method of measuring the specific gravity of urine. The refractometer method is the gold standard for determining the specific gravity of urine. However, the refractometer has the major disadvantage of requiring daily calibration and not being adapted for home monitoring.

A third urinalysis method for specific gravity, the falling drop method, like the urinometer, is a direct measurement of urine specific gravity. In accordance with this method, a drop of urine is introduced into each of a series of columns that are filled with solvent mixtures of increasing and known specific gravity. Prior to development of the refractometer, this technique had the advantage of requiring only a few drops of test sample to conduct a specific gravity assay. The falling drop method, however, never achieved widespread use in routine urinalysis because of the obvious time requirements in setting up such a system and the inability for an individual to perform the assay at home.

Each of the above described instrument-based specific gravity assay methods has disadvantages, whereby none of the assay methods are particularly well suited to performing specific gravity assays outside of the physician's office or laboratory.

Consequently, reagent impregnated test strips have been developed to allow specific gravity determination to be performed at home. Such test strips measure specific gravity indirectly, the test strip changing color in response to the ionic strength of the urine sample.

In particular, these test strips measure sodium concentration, which is the main contributor to specific gravity. Sodium ions are captured using polyelectrolytes and hydrogen ions are released. These hydrogen ions are then detected with a pH indicator which shows a change of color.

However, the presently available commercial test strips have been known to have issues in their performance due to their sensitivity to changes in the pH of the urine. This makes the tests inherently difficult to interpret as the values are disperse. For example, the article *"*Dipstick measurements of urine specific gravity are unreliable" by de Buys Roessingh et al compared different methods and concludes that only refractometry gives reliable urine specific gravity results contrary to dipstick measurements (i.e. test strips).

Several solutions have been suggested to improve the specific gravity detection.

US 4,318,709 discloses the use of weakly acidic or basic polyelectrolytes which have been at least 50% neutralized with a base or an acid. Depending on the ionic strength of the test solution, an intramolecular pH change may occur in the polymer, the degree of which is a barometer of ionic strength. A pH indicator such as a pH sensitive compound reflects the pH change (or lack thereof) instigated by the sample ionic strength.

US5,403,744 discloses a composition for determining specific gravity comprising a strong polyelectrolyte, an indicator and buffered at a pH of 3 or less.

US 4,376,827 discloses a composition for determining specific gravity comprising a strongly acidic or strongly basic polyelectrolyte and a buffer substance capable of providing a pH of about 5.5 and an indicator means.

EP0023631 discloses a reagent for determining the specific gravity. In addition to a strongly acidic or strongly basic polyelectrolyte polymer, it contains a buffer substance that ensures a pH value of at least 5.5 and a pH indicator.

### SUMMARY

In contrast to the prior art, and in contrast to the presently available commercial test strips, it is an aim of the present disclosure to provide reduced variability and increased sensitivity in the measurement of urine specific gravity.

It is also an aim of the disclosure to provide a rapid, accurate and reliable determination of the specific gravity of urine using a test strip which could be performed at home.

To that aim, the disclosure relates to a method to manufacture a test strip for measuring specific gravity of a urine sample, the method comprising successively:
- providing a polyelectrolyte in a first solution, the polyelectrolyte being poly(methyl vinyl ether-alt-maleic anhydride),
- adding to the first solution a base, to neutralize the polyelectrolyte and to bring the first solution at a pH between 10 and 11,
- adding to the first solution a buffering agent to bring and maintain the first solution at a pH between 7.0 and 7.5,

- dipping a paper strip into the first solution,
- dipping the paper strip into a second solution comprising a pH sensitive color indicator.

The disclosure also relates to a method to manufacture a test strip for measuring specific gravity of a urine sample, the method comprising successively:
- providing a polyelectrolyte in a first solution, the polyelectrolyte being notably a polyacid,
- adding to the first solution a base, to neutralize the polyelectrolyte and to bring the first solution at a pH between 10 and 11,
- adding to the first solution a buffering agent to bring and maintain the first solution at a pH between 7.0 and 7.5,
- dipping a paper strip into the first solution,
- dipping the paper strip into a second solution comprising a pH sensitive color indicator.

In an embodiment, the method further comprises drying the paper strip after each dipping.

In an embodiment, the base is sodium hydroxide (NaOH).

In an embodiment, the base is a mixture of sodium hydroxide (NaOH) and potassium hydroxide, preferably at a mass ratio between 2.6 and 3.5.

In an embodiment, the neutralization is made by titration of the polyelectrolyte until the first solution reaches a pH between 10 and 11.

In an embodiment, the neutralization is made by a one pot synthesis.

In an embodiment, the polyelectrolyte is at least about 80% neutralized, preferably more than 85% neutralized.

In an embodiment, the buffering agent is Tris(hydroxymethyl)aminomethane hydrochloride (Tris HCl), in particular a solution at 1 M (mole/L) of Tris HCl.

In an embodiment, the pH sensitive color indicator is bromothymol blue.

The disclosure also relates to a test strip for determining the specific gravity of a urine sample urine manufactured by the method as described above, the test strip determining the specific gravity of the urine sample based on the intensity of color change of the pH sensitive color indicator.

In an embodiment, the test strip further comprises a pH test pad.

The disclosure also relates to a cartridge for a urine optical analysis device, the cartridge comprising a plurality of chambers arranged next to one another in a right circular cylinder shape of at least 80% of a circle with a diameter comprised between 3 and 10 cm, wherein at least a chamber comprises a test strip as described above.

The cartridge further comprises a pH measuring strip arranged in at least a chamber.

The disclosure also relates to a urine optical analysis device urine comprising:
- a cartridge as described above,
- a station, configured to be positioned on a wall of a toilet bowl, the station comprising:
   + a case comprising a compartment in which the cartridge is at least partially received,
   + an injector, configured to inject fluid on the test strip,
   + an analyzer to analyze a change of color of the test strip due to fluid from the fluid sample.

In an embodiment, the analyzer includes a light source and a light sensor, configured to emit and receive light.

### BRIEF DESCRIPTION OF THE DRAWINGS

These features and advantages of the disclosure will appear more clearly upon reading the following description, provided solely as a non-limiting example, and done in reference to the appended drawings, in which:
Figure 1 shows the overall setup of an analysis device for urine analysis according to an embodiment, as installed on a surface of a toilet bowl,
Figure 2 shows an exploded view of an embodiment of an analysis device, in which the station and the cartridge are visible,
Figure 3 shows a detailed view of a cartridge according to an embodiment,
Figure 4 shows a sectional view of an embodiment of a cartridge and a station, at the location of an optical analyzer of the station,
Figure 5 shows a top view of a test strip according to an embodiment,
Figure 6 shows a side view of the test strip of Figure 5,
Figure 7 shows a chemical representation of the polyelectrolyte used in the method according to the present disclosure,
Figure 8 represents a method of manufacturing a test strip for measuring specific gravity of a urine sample according to the present disclosure,
Figure 9 shows a graph representing normalized intensities as a function of specific gravity:
   - with a test strip manufactured with a reference method,
   - with a test strip manufactured with a method according to the present disclosure with NaOH as a base, and
   - with a test strip manufactured with a method according to the present disclosure with a mixture of NaOH and KOH as a base.
Figure 10 shows a graph representing normalized intensities as a function of specific gravity with a test strip manufactured with a method according to the present disclosure without the buffering step and with different urine pH.
Figure 11 shows two graphs representing normalized intensities as a function of specific gravity:
   - with a test manufactured with a reference method with and without the buffering step (a) and
   - with a test strip manufactured with a method according to the present disclosure with and without the buffering step (b).
Figure 12 shows a graph representing normalized intensities as a function of specific gravity with a test strip manufactured with a method according to the present disclosure using NaOH as a base, using urine containing different ion compositions.
Figure 13 shows a graph representing normalized intensities as a function of specific gravity with a test strip manufactured with a method according to the present disclosure using a mixture NaOH/KOH as a base, in presence of different potassium concentrations.

### DETAILED DESCRIPTION

The present description introduces different examples of a cartridge usable with a station as disclosed in document WO2021/175909 and WO2021/175944, hereafter referred to as WO'909 and WO'944. Variations of the stations are presented in any of WO2023036805, WO2023036806, WO2023036808, WO2023036809, hereafter referred to as WO'80X.

The next paragraphs explain the overall principle of a device for urine analysis, but all the details of WO'909 and WO'933 (and also any of the above-mentioned PCT filings) are applicable.

### OVERALL DESCRIPTION OF THE STATION AND THE CARTRIDGE

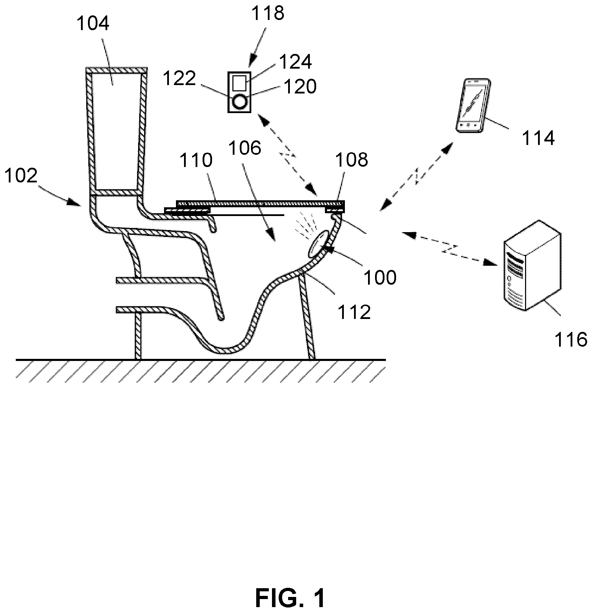

Figure 1 schematically illustrates an analysis device 100 (referred to as the "device 100") for urine analysis as set up in a toilet 102. Toilet 102 usually comprises a water tank 104, a bowl 106, a seat 108 and a seat cover 110. The analysis device 100 is arranged in a removable manner in the toilet 102. For example, the analysis device 100 may be easily removed from the toilet to replace a cartridge and then arranged again in the toilet 102. The analysis device 100 is arranged on an internal wall 112 of the bowl 106 of the toilet. The analysis device 100 is placed so that it is usually under a urine stream from a user, such that when a user urinates (typically in a seated position), urine contacts with the analysis device 100. The analysis device 100 may communicate remotely with a remote entity, such as smartphone 114 or a server 116.

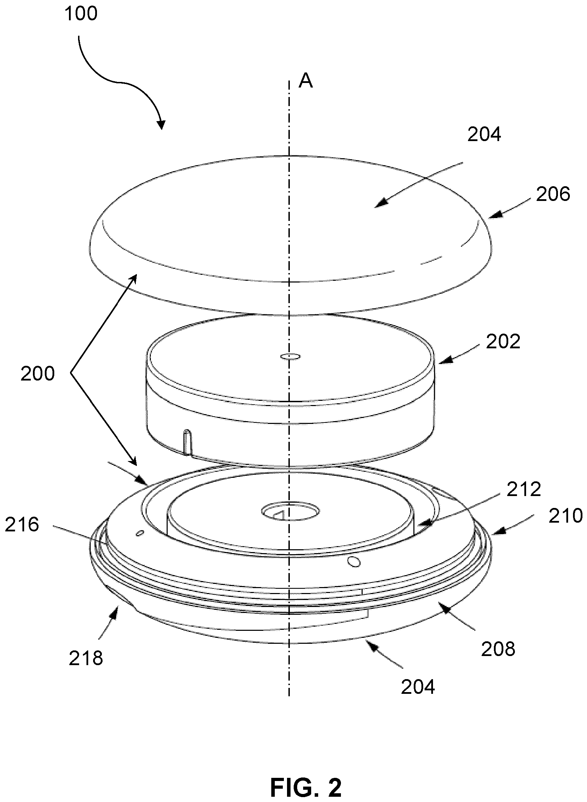

As illustrated in more detail on Figure 2, the analysis device 100 comprises a station 200 and a cartridge 202, mounted in a removable manner from the station 200. Station 200 may comprise a case 204 which may include two shells 206, 208. Case 204 lodges therein a urine testing assembly. Station 200 comprises an annular or ring-shaped compartment 212, located inside the case 204, arranged around a rotation axis A. The annular compartment 212 is configured to receive at least partially the cartridge 202 mounted in a rotatable manner around the rotation axis A (once in position in the annular compartment 212). The cartridge 202 comprises a plurality of test supports which each comprise at least one urine reagent, for instance a dry reagent, the plurality of test supports being arranged along a circle or a circular arc around the rotation axis A. In an embodiment, the test supports are test strips. The test supports may be enclosed, for example individually enclosed, in a chamber.

The annular compartment 212 typically extends around 360° and forms a groove configured to receive at least partially the cartridge 202.

Station 200 comprises a collection opening 218, located for example on shell 208. The collection opening 218 collects urine flowing on the surface of the case 204. A drain opening (not illustrated) is also included to drain the liquid out of the device 100.

The case 204 may have a diameter, in a direction perpendicular to the rotation axis A, comprising between 50 mm and 150 mm.

The test assembly may comprise a pump, an injector and an analyzer. The pump sucks urine from the collection opening 218, then the injector injects the urine on one or more test supports of the cartridge and the analyzer obtains some values of properties (e.g., physical/chemical properties, such as the color) of the test supports after it has contacted the urine. In one embodiment, the analyzer is an optical analyzer configured to analyze optical properties of the test support. The injector and the cartridge may move relatively to each other so that the injector can open (e.g., pierce) the chamber, for example with a needle or needle-like device.

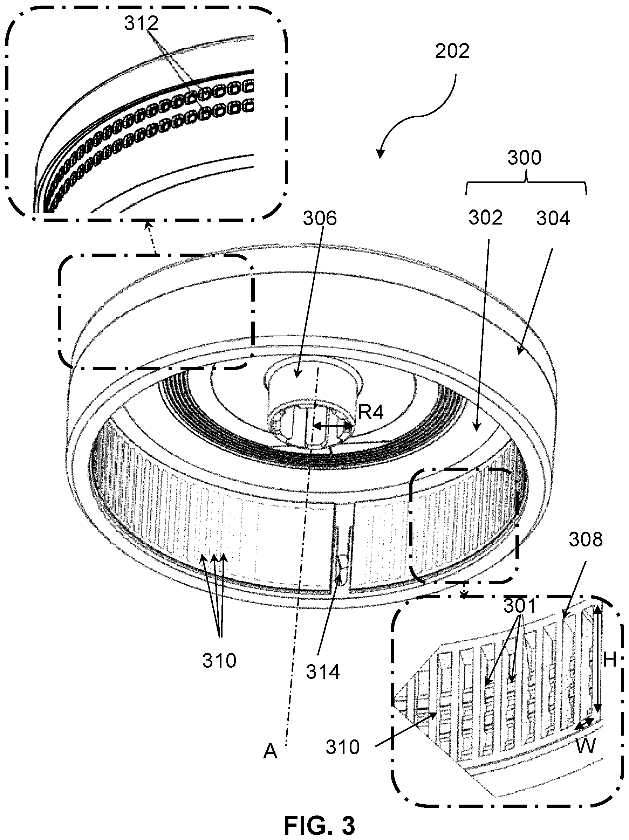

Figure 3 shows an exploded view of the cartridge 202. The cartridge 202 comprises at least a test support 301, notably several test supports 301 configured to receive urine from the injector. Each test support 301 contains a urine reagent that reacts in a specific way when in contact with urine. The cartridge 202 comprises a rotatable support 300, configured to be driven in rotation by the station 200. In normal use of the cartridge 202 and the device 100, the test supports 301 remain attached to the rotatable support and do not move with respect to the latter.

In an embodiment, the rotatable support 300 has a right circular cylinder shape of at least 80% of a hollow cylinder shape extending annularly around an axis which is, when the cartridge 202 is mounted in the station 200, the rotation axis A. Each test support 301 may be a test strip. The rotatable support 300 may comprise an annular portion 302 and a cylindrical portion 304, which extends from an outermost radial extremity of the annular portion 302. The cylindrical portion 304, when in use, is lodged inside the annular chamber 212. The test supports 301 are positioned along the cylindrical portion 304, so that they can be selectively and/or successively scrolled in front of the injector and the analyzer. For instance, the test supports 301 are part of a holder 308, which comprises several chambers 310, separated from each other along a perimeter around the axis A. At least a test strip is received in a chamber 310. Advantageously, a single test strip is received in a chamber 310. In particular, each chamber 310 comprises a single test strip.

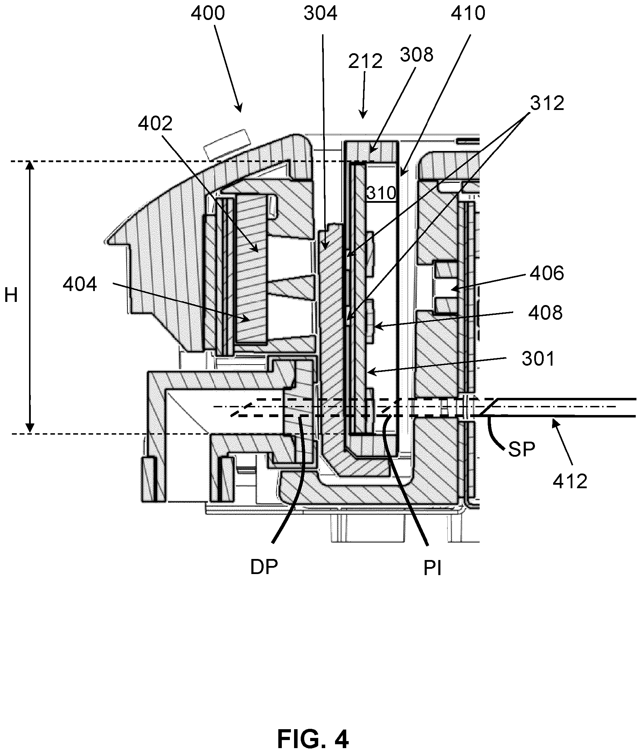

The plurality of chambers 310 are arranged next to one another in a right circular cylinder shape of at least 80% of a circle. To allow light to go through, the holder 308 includes at least one aperture 312 per chamber 310 (represented in the upper left zoom where the rotatable support is shown as transparent). The chambers 310 are all at an equal distance of the rotation axis A, so that the injector can selectively inject urine after the desired chamber is positioned at a desired location facing the injector. The injector may translate towards the chamber 310 and pierce a lid closing the chamber 310 (visible on Figure 4). A drain opening 314 is provided in the rotatable support 300 to allow evacuating urine from the injector to the outside of the device 100.

Each chamber 310 may have a maximum dimension, e.g., a height H, which is less than 10% more than the maximal expanse of the test support, as it will be explained below. In particular, each chamber 310 may have a height along the rotation axis A lower than 2 cm, notably lower than 1,5 cm.

Each chamber 310 may have a transversal width W, orthogonally to the rotation axis A, comprised between 1 mm and 6 mm.

The annular portion 302 of the rotatable support 300 remains outside of the annular chamber 212 to strengthen the cylindrical portion and/or to drive in rotation the cartridge 202. To that end, the annular portion 302 may include a mechanical coupling 306, which cooperates with a shaft of the station 200.

The dimensions relative to the cartridge 202 are disclosed in WO'909, WO'933, and WO'80X. The maximum dimension of the device 100 transversal to the rotational axis A is less than 15 cm, even less than 10 cm. The maximum dimension of the device along the rotation axis A is less than 5 cm.

Figure 4 shows in more detail the interaction between the cartridge 202 and the station 200 when or after the injector is activated. The analyzer 400 comprises at least one light source 402, 404 (e.g., two) and at least one optical sensor 406. Light travels from the light source 402, 404 to the optical sensor 406 while passing through the cartridge 202 and in particular the cylindrical portion 304, the aperture 312 of the holder 308, the test support 301 and thus the reagent 408.

In one embodiment, the analyzer 400 is configured to measure the absorbance of a part of the test supports 301 (notably the test line and/or control line of a test strip as it will be explained below). The absorbance is detected by the light source (for example a led) which may pass light through the strip, and the optical sensor which receives the spectrum with about ten wavelengths.

In a variant, the light sensor is a camera able to detect a change of color, notably a change of intensity of color, of a part of the test supports 301 (notably the test line and/or control line of a test strip as it will be explained below). The camera may detect a color in RGB values for example.

The injector includes an injection end 412 (for example a needle), which can be moved between several positions, which are represented in dash lines in Figure 4. In a standby position SP, the injection end 412 is outside the cartridge 202 (in an innermost position), so that the cartridge 202 can rotate freely in the annular housing 212; in an injection position IP, the injection end 412 has pierced the lid 410 to access the inside of the chamber 310 and may inject some urine on the test support 301; in the drain position DP, the injection end 412 is able to evacuate urine through the drain opening 314 of the rotatable support 300.

In position SP, the injector is located radially inward the annular chamber. This allows to maximize the radius of the annular chamber while minimizing the size of the station 200.

### OVERALL DESCRIPTION OF THE TEST STRIP

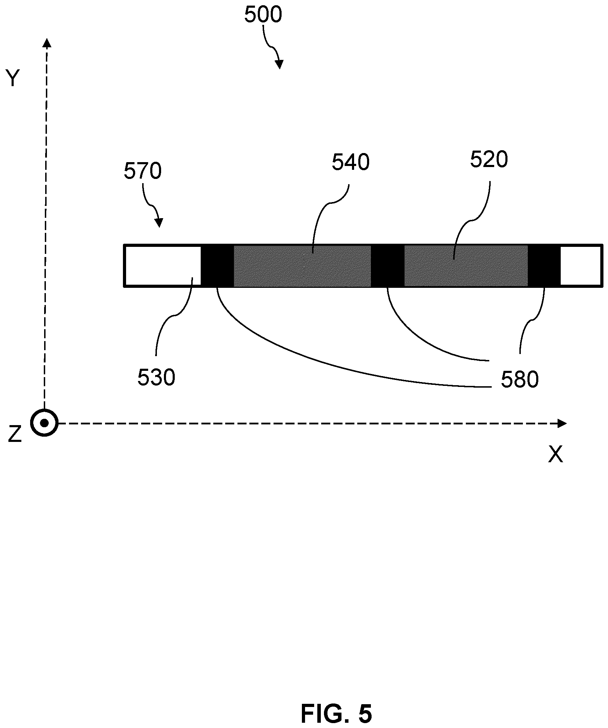

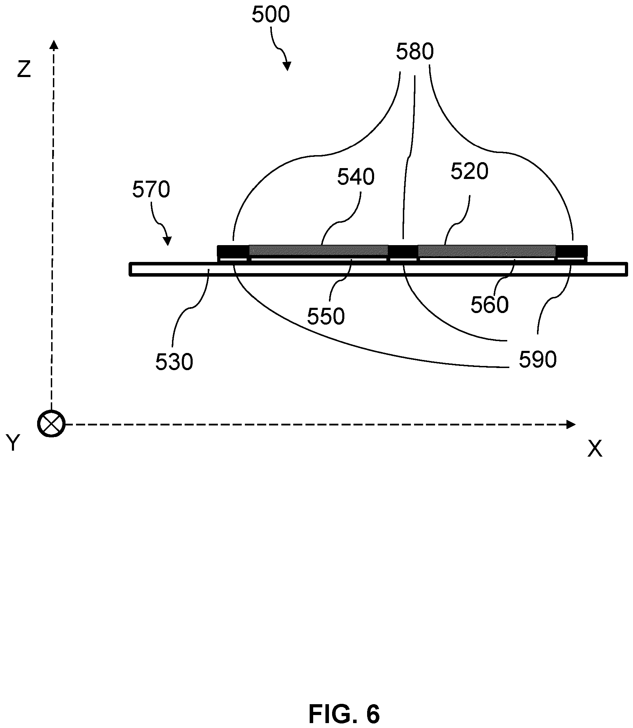

The test support 301 may be a test strip 500. As visible on Figure 5 and Figure 6, the test strip 500 extends mainly along a longitudinal direction X. In one embodiment, the test strip 500 has a generally parallelepipedic shape. The test strip 500 may present a width inferior to 5 mm, notably between 0.5 mm and 3 mm in a transversal direction Y, orthogonally to the longitudinal axis X. The test strip 500 may present a length inferior to 20 mm, notably between 10 mm and 15 mm, along the longitudinal axis X. The test strip 500 presents a thickness along an elevation axis Z that varies along the strip depending on its components. The test strip 500 comprises different components and materials that absorb and/or react to urine, and notably to the sodium in the urine or the pH of the urine.

To that end, the test strip 500 comprises a test pad 520 configured to measure a specific gravity of the urine (and called specific gravity test pad 520) and a backing pad 530. The test strip 500 may further comprise a test pad 540 configured to measure a pH of the urine (and called a pH test pad 540).

The backing pad 530 may be made of Whatman CF3 paper.

The specific gravity test pad 520 is fixed to the backing pad 530 for example with at least an adhesive tape 550. The adhesive tape 550 is in particular a double-sided adhesive. The adhesive tape 550 is for example an acrylic based adhesive.

The pH test pad 540 is fixed to the backing pad 530 for example with at least an adhesive tape 560. The adhesive tape 560 is in particular a double-sided adhesive. The adhesive tape 560 is for example an acrylic based adhesive.

An extremity 570 of the test pad 500 along the longitudinal axis X, in particular the extremity of the backing pad 530, is configured to receive a urine sample. In particular, the extremity 570 is configured to face the injector end 412 when the test strip 500 is arranged in a chamber 310 of the cartridge 202 and placed inside the analysis device 100. The extremity 570 is configured to receive the urine injected by the injector on the test strip 500.

The specific gravity test pad 520 may extend on a length along the longitudinal axis X inferior to 4 mm.

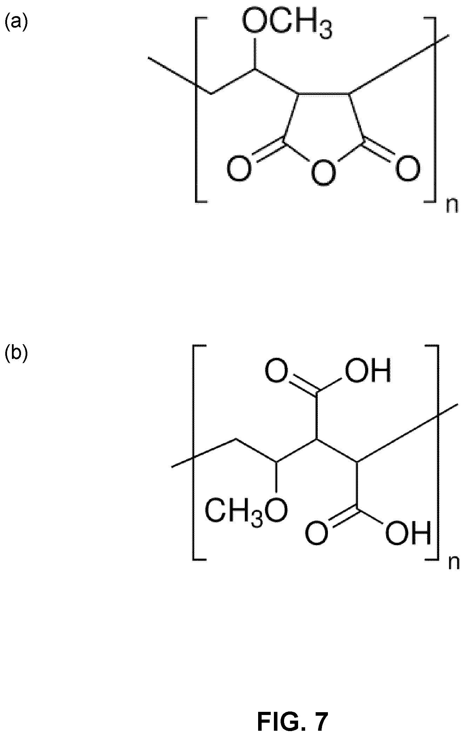

The specific gravity test pad 520 comprises a carrier matrix impregnated with a reagent composition comprising a polyelectrolyte, a pH sensitive color indicator and a buffering agent. The polyelectrolyte is in particular a partially neutralized poly(methyl vinyl ether-alt-maleic acid), as represented on Figure 7 (b). The pH sensitive color indicator may be bromothymol blue. A manufacturing method of the specific gravity test pad 520 will be disclosed below.

The polyelectrolyte used in the present disclosure is a polyacid. The polyacid is neutralized with a base or a mixture of bases. During neutralization, several hydrogen ions in the carboxylic groups are replaced with sodium or sodium and potassium depending on the base used. When a solution of a known specific gravity is added to the reagent pad containing the polyelectrolyte, the degree of ionization of the polyelectrolyte changes causing a release of H+ ions and change in pKa of the polyelectrolyte. This release of H+ causes a change in the pH on the specific gravity test pad 520 which is measured using the pH indicator. In the presence of buffer in the recipe, the surface pH of the specific gravity test pad 520 is controlled by the buffer and any change in pH on the specific gravity test pad 520 due to specific gravity is measured as a deviation of pH from the buffer pH.

The pH test pad 540 is arranged next to the specific gravity sample pad 520, in particular between the extremity 570 and the specific gravity pad 520 along the longitudinal axis X. The pH test pad 540 may extend on a length along the longitudinal axis X inferior to 4 mm.

A conventional pH test pad 540 may be used here.

Referring again to Figure 4, each test pad 520, 540 is configured to face the light sources 402, 404 on one side and the optical sensor 406 on the other side when the test strip 500 is arranged in a chamber 310 of the cartridge 202 and placed inside the analysis device 100.

The test strip 500 may further comprise at least an optical mask 580, notably three optical masks 580. Each mask 580 is fixed to the backing pad 530 with at least an adhesive tape 590. Each optical mask 580 enables to prevent light leaks and improve the sensitivity of the optical sensor and the accuracy of the measurements.

The test strip 500 disclosed here may be used with the cartridge 202 previously disclosed and the urine analysis device as it will be described below. In particular, at least a test strip 500 is received in a chamber 310 of the cartridge 202. They provide a cost efficient, manufacturing efficient, easily adaptable, and mass-production suitable solution to improve the quality of the optical analysis and in particular the specific gravity measurements.

When the test strip 500 does not comprise a pH test pad 540, the cartridge 202 may further comprise a pH measuring strip arranged in at least a chamber 310, different from the chamber in which the test strip 500 is received.

### MANUFACTURING METHOD OF THE TEST STRIP

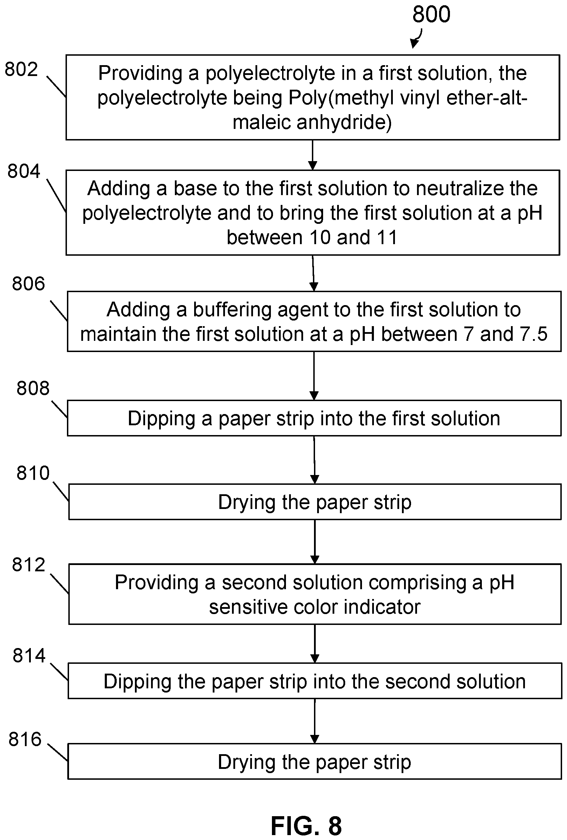

A method 800 to manufacture a specific gravity test pad 540 will now be described in reference to Figure 8.

In step 802, a polyelectrolyte is provided in a first solution. The polyelectrolyte is in particular a polyacid. The polyelectrolyte is in particular poly(methyl vinyl ether-alt-maleic anhydride), represented on Figure 7 (a). For example, the poly(methyl vinyl ether-alt-maleic anhydride) may be provided as a powder and is then dissolved into an aqueous solvent to form the first solution. At the contact of water, poly(methyl vinyl ether-alt-maleic anhydride) becomes poly(methyl vinyl ether-alt-maleic acid) as represented on Figure 7 (b).

In step 804, called neutralization step 804, a base is added to the first solution to neutralize the polyelectrolyte and to bring the first solution at a pH between 10 and 11.

By neutralization of the polyelectrolyte, it is understood that at least a part of the ionizable groups are neutralized. In particular, one or more of the carboxyl groups are neutralized.

The polyelectrolyte is at least about 80% neutralized, preferably more than 85% neutralized. This means that at least 80% of the carboxyl groups are neutralized, preferably at least 85%.

In an embodiment, the base is sodium hydroxide (NaOH).

In a variant potassium hydroxide, the base is a mixture of sodium hydroxide (NaOH) and potassium hydroxide (KOH).

In an embodiment, the mass ratio between NaOH and is between 2.6 and 3.5, for a poly(methyl vinyl ether-alt-maleic anhydride) provided by Sigma-Aldrich (ref 416339). When supplied by another manufacturer, the mass ratio should be adapted accordingly.

The polyelectrolyte, which is a carboxylic acid, reacts with the strong bases that are NaOH and KOH to produce carboxylate salts:

RCOOH + NaOH(aq) → RCOO-Na+(aq) + H2O

RCOOH + KOH(aq) → RCOO-K+(aq) + H2O

In an embodiment, the neutralization is made by titration of the polyelectrolyte. In particular, the titration is made until pH between 10 and 11.

In a variant, the neutralization is made by a one pot synthesis. One pot synthesis is a method in which the chemical compounds are mixed together in a single vessel and allowed to react, rather than conducting the reaction in a sequence of separate stages. In particular, one pot synthesis is used when the quantity of base to use so that the first solution reaches a pH between 10 and 11 is known.

In step 806, called buffering step 806, a buffering agent is added to the first solution, after the polyelectrolyte neutralization, to bring the first solution at pH between 7.0 and 7.5. The buffering agent enables to then maintain the first solution at pH between 7.0 and 7.5. At this step, the first solution comprises the polyelectrolyte and the buffer.

In particular, the buffer is Tris(hydroxymethyl)aminomethane hydrochloride (Tris HCl). Preferably, the buffer is a solution at 1 M (mole/L) of Tris HCl.

It is noted that since there is no sodium in this buffer solution, the neutralization of the polyelectrolyte carried out previously is not disrupted.

After the buffering step, the concentration of polyelectrolyte in the first solution may be comprised between 0.020 g/mL and 0.025 g/mL, notably around 0.023 g/mL, for a poly(methyl vinyl ether-alt-maleic anhydride) provided by Sigma-Aldrich (ref 416339).

When supplied by another manufacturer, the polyelectrolyte concentration should be adapted according to the polyelectrolyte's characteristics (IR spectrum), its viscosity, etc.

In step 808, a paper strip is dipped into the first solution. At this step, the first solution comprises the polyelectrolyte, the base, and the buffer.

In one embodiment, the paper strip is made of Whatman CF3 backing with reagent pads made of CF3 paper.

In particular, the paper strip is dipped into the first solution during at least 3 to 5 minutes.

In step 810, the paper strip is then dried. For example, the paper strip is dried in an oven for at least 60 minutes.

In step 812, a second solution is provided. The second solution comprises a pH sensitive color indicator. The pH sensitive color indicator is for example bromothymol blue. The second solution may comprise an organic solvent.

In one embodiment, the bromothymol blue is present at a concentration of 0.001 g/mL in the second solution.

In step 814, the paper strip is dipped into the second solution comprising a pH sensitive color indicator. In particular, the paper strip is dipped into the second solution for at least 3 to 5 minutes.

In step 816, the paper strip is then dried. For example, the paper strip is dried in an oven for at least 60 minutes.

The paper strip after step 816 of the manufacturing method 800 is the specific gravity test pad 520 as disclosed previously in relation to Figure 6.

### EXPERIMENTS

The disclosure is further illustrated by the following experiments.

### Experiment #1:

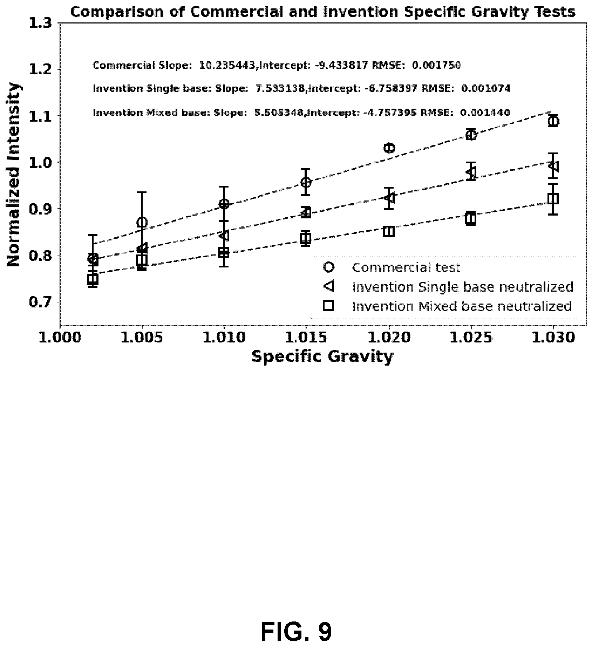

In reference to Figure 9, experiment #1 compares specific gravity measurements with a test strip manufactured:
- with a reference method (dots),
- with a test strip manufactured with a method according to the present disclosure with NaOH as a base (triangle), and
- with a test strip manufactured with a method according to the present disclosure with a mixture of NaOH and KOH as a base (square).

The reference method comprises dipping a paper strip into a first solution comprising poly(methyl vinyl ether-alt-maleic anhydride) neutralized to pH 8, followed by dipping the paper strip into a second solution comprising bromothymol blue, which is pH sensitive color indicator. The term "reference method" is used here only to designate a method used for comparison with the method according to the present disclosure.

The method according to the present disclosure here comprises dipping a paper strip into a first solution comprising poly(methyl vinyl ether-alt-maleic anhydride) neutralized to pH between 10 to 11 then buffed at pH between 7.0 and 7.5, followed by dipping the paper strip into a second solution comprising bromothymol blue, which is pH sensitive color indicator.

Urine pH is uncontrolled and varies between 6 and 7.

Urine's specific gravity varies between low specific gravity of 1,00 to a high specific gravity of 1,03.

The potassium concentration is lower than 40 mM.

The graphs on Figure 9 represent normalized intensities as a function of the urine's specific gravity.

Figure 9 shows a better linearity for the two methods according to the disclosure than the reference method, despite presenting a lower slope.

### Experiment #2:

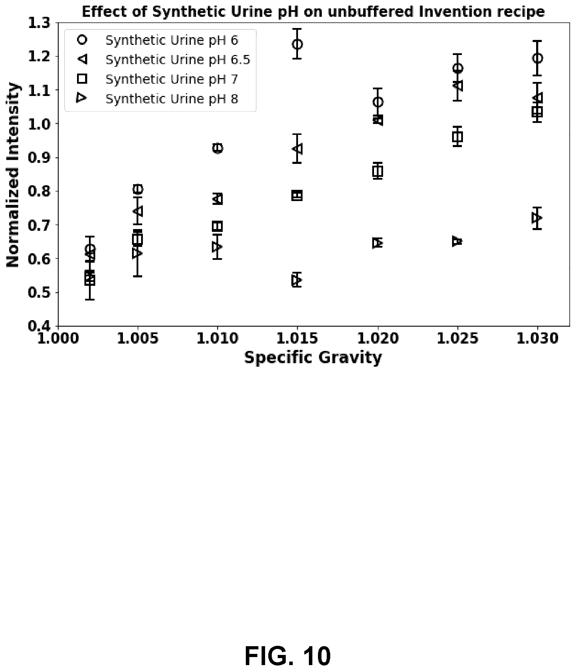

In reference to Figure 10, experiment #2 compares specific gravity measurements with a test strip manufactured with a method without a buffering step and with synthetic urine at respectively pH 6, 6.5, 7 and 8.

The method here comprises dipping a paper strip into a first solution comprising poly(methyl vinyl ether-alt-maleic anhydride) followed by dipping the paper strip into a second solution comprising bromothymol blue, which is pH sensitive color indicator.

Figure 10 shows that the best linearity is achieved with pH 7. Hence, the method according to the present disclosure comprises adding a pH 7.0 to 7.5 buffer in the polyelectrolyte preparation step.

### Experiment #3:

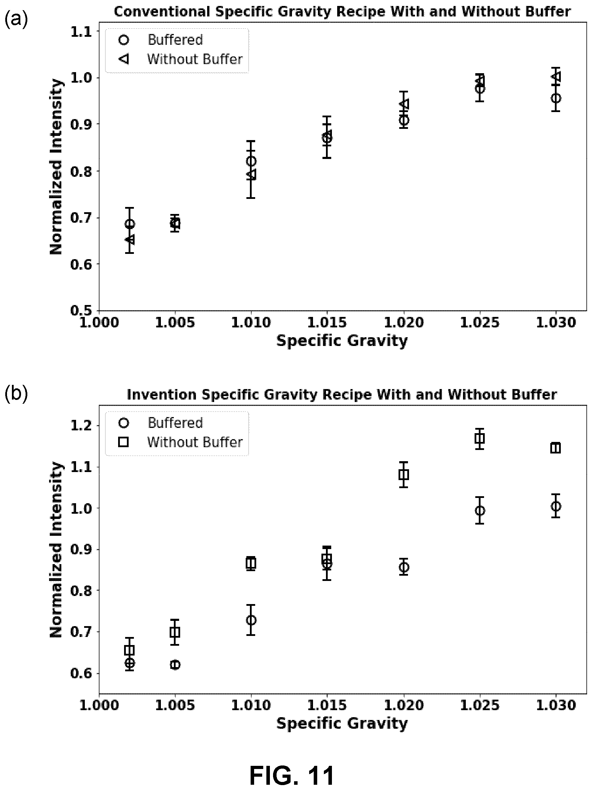

In reference to Figure 11, experiment #3 compares specific gravity measurements:
- (a) with a test strip manufactured with a reference method with and without a buffering step at a pH 7, and
- (b) with a test strip manufactured with a method 800 comprising steps 802, 804, 806, 808 and 814 and with a method comprising steps 802, 804, 808 and 814, but not comprising the buffering step 806 of method 800.

The reference method (a) comprises dipping a paper strip into a first solution comprising poly(methyl vinyl ether-alt-maleic anhydride) neutralized to pH 8, with and without the buffering step, followed by dipping the paper strip into a second solution comprising bromothymol blue, which is pH sensitive color indicator.

The method (b) here comprises dipping a paper strip into a first solution comprising poly(methyl vinyl ether-alt-maleic anhydride) neutralized to pH between 10 to 11, with and without the buffering step 806, followed by dipping the paper strip into a second solution comprising bromothymol blue, which is pH sensitive color indicator.

A synthetic urine at pH 6.5 here is used.

Figure 11 (b) shows lower errors and a plateau above specific gravity of 1.025 in the presence of buffer compared to the reference test in Figure 11 (a) which shows a saturation at specific gravity of 1.020 with or without buffer.

From Figure 11 (b) the impact of the presence or absence of buffer is prominent compared to Figure 11 (a). The amplitude of the response curve in Figure 11 (b) without buffer is larger than with buffer but the errors are globally improved, notably at lower specific gravities, with buffer than without buffer.

Further, it is anticipated that the presence of the buffer would reduce the impact of urine pH on the test.

### Experiment #4:

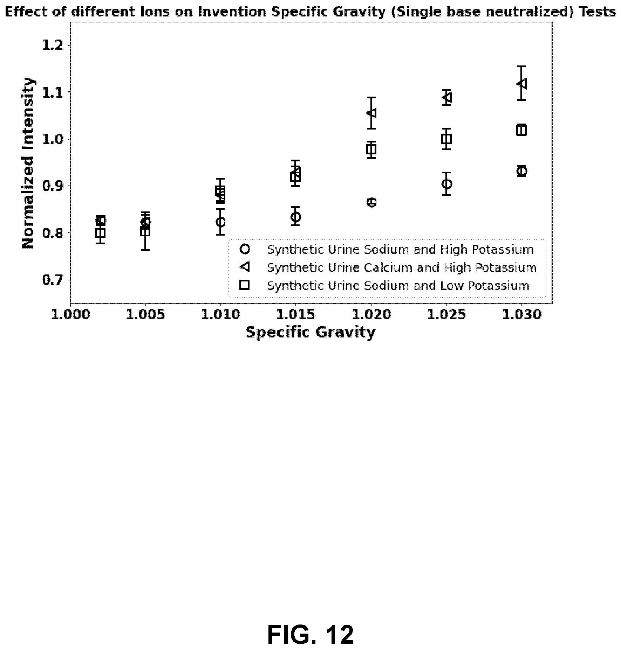

In reference to Figure 12, experiment #4 compares specific gravity measurements with a test strip manufactured with a method according to the present disclosure in presence of different ions compositions in the urine:
- sodium and high potassium concentration (around 70 mM K+);
- calcium instead of sodium and high potassium; and
- calcium and low potassium (<2mM K+).

The method according to the present disclosure here comprises dipping a paper strip into a first solution comprising poly(methyl vinyl ether-alt-maleic anhydride) neutralized to pH between 10 to 11 then buffered at pH between 7.0 and 7.5, followed by dipping the paper strip into a second solution comprising bromothymol blue, which is pH sensitive color indicator.

The polyelectrolyte is neutralized here with NaOH.

Figure 12 shows that the specific gravity measurements made with test strips manufactured with a method according to the present disclosure show low sensitivity to the ion composition (notably the presence of potassium and calcium) for the same specific gravity which is acceptable. In particular, if urine has calcium and potassium but no sodium, the test strip would still respond to specific gravity.

### Experiment #5:

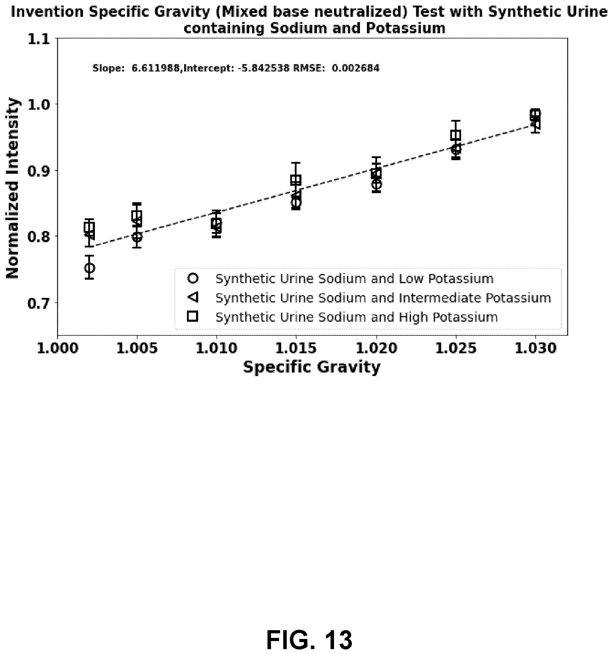

In reference to Figure 13, experiment #5 compares a specific gravity measurement with a test strip manufactured with a method according to the present disclosure in the presence of low, intermediate and high concentrations of potassium (<2mM K+, around 40 mM K+ and around 70 mM K+ respectively).

The method according to the present disclosure here comprises dipping a paper strip into a first solution comprising poly(methyl vinyl ether-alt-maleic anhydride) neutralized to pH between 10 to 11 then buffered at pH between 7.0 and 7.5, followed by dipping the paper strip into a second solution comprising bromothymol blue.

It is known that presence of a high concentration of potassium reduces the response compared to the response in urine with low potassium (less amplitude between low and high specific gravities).

To compensate for this effect, the polyelectrolyte is neutralized with a ratio of bases KOH/NaOH. Indeed, Figure 13 shows that the mixture of sodium hydroxide and potassium hydroxide enables to reduce the potassium sensitivity for the specific gravity measurements compared to using only sodium hydroxide.

### OPERATION OF THE STATION AND THE TEST STRIP

The operation of station 200 and the test strip 500 will now be described.

Station 200 is installed in toilets 102, as illustrated on Figure 1. A cartridge 202 is mounted in a removable manner in the station 200.The cartridge 202 comprises at least a test strip 500 as described above arranged in a chamber 310. Advantageously, the cartridge 202 further comprises a pH measuring strip arranged in at least a chamber 310.

An individual may urinate on station 200. A part of the urine stream is collected through the collection opening 218. Referring to Figure 4, the injector is initially in the standby position SP and the injection end 412 is outside the cartridge 202 (in an innermost position), so that the cartridge 202 can rotate freely in the annular housing 212. In particular, the test strip 500 is placed in front of the injector.

Then, the injector switches to the injection position IP and the injection end 412 pierces the lid 410 to access the inside of the chamber 310 and injects some urine on the extremity 570.

The urine and the sodium ions present in the urine flow through the test strip 500, in particular in the direction of the specific gravity test pad 520 and eventually to the pH test pad 540 thanks to the backing pad 530.

The specific gravity test pad 520 shows a change of color due to the pH sensitive color indicator, notably bromothymol blue, in response to urine's specific gravity.

The pH test pad 540 shows a change of color in response to the urine pH.

The station 200 then carries out an optical analysis. To that end, the light source 402, 404 illuminates the test pad(s) 520, 540 and eventually the reference pad 540. Light travels from the light sources 402, 404 to the optical sensor 406 while passing through the cartridge 202 and in particular the cylindrical portion 304, the aperture 312 of the holder 308, the test strip 500 and in particular the test pad(s) 520, 540.

The optical sensor 406 acquires optical data via the light emitted from the test strip 500. In particular, the optical sensor 406 analysis the variation of color and determine a specific gravity of the urine sample and eventually the pH of the urine sample.

The optical sensor 406 may use the measured pH to verify that urine pH is within a range medically considered normal, for example between pH 5,5 and 7.

The station 200 may communicate remotely the determined specific gravity, and eventually pH, with a remote entity, such as smartphone 114 or a server 116. In a variant, the station 200 may communicate an indicator as "low", "normal" or "high" representative of the measured specific gravity compared to an expected specific gravity. The individual may therefore easily and regularly monitor the specific gravity of its urine.

## Claims

1. A method (800) to manufacture a test strip (500) for measuring specific gravity of a urine sample, the method comprising successively:
- providing (802) a polyelectrolyte in a first solution, the polyelectrolyte being poly(methyl vinyl ether-alt-maleic anhydride),
- adding (804) to the first solution a base to neutralize the polyelectrolyte and to bring the first solution at a pH between 10 and 11,
- adding (806) to the first solution a buffering agent to bring and maintain the first solution at a pH between 7.0 and 7.5,
- dipping (808) a paper strip (520) into the first solution,
- dipping (814) the paper strip into a second solution comprising a pH sensitive color indicator.

2. The method according to claim 1, further comprising at least one of: drying (810) the paper strip after the dipping (806) into the first solution and drying (816) the paper strip after the dipping (814) into the second solution.

3. The method according to claim 1 or 2, wherein the base is sodium hydroxide (NaOH)

4. The method according to claim 1 or 2, wherein the base is a mixture of sodium hydroxide (NaOH) and potassium hydroxide (KOH), preferably at a mass ratio between 2.6 and 3.5.

5. The method according to any of claims 1 to 4, wherein the neutralization is made by titration of the polyelectrolyte until the first solution reaches a pH between 10 and 11.

6. The method according to any of claims 1 to 4, wherein the neutralization is made by a one pot synthesis.

7. The method according to any of claims 1 to 6, wherein the polyelectrolyte is at least about 80% neutralized, preferably more than 85% neutralized.

8. The method according to any of claims 1 to 7, wherein the buffering agent is Tris(hydroxymethyl)aminomethane hydrochloride (Tris HCl), in particular a solution at 1 M (mole/L) of Tris HCl.

9. The method according to any of claims 1 to 8, wherein the pH sensitive color indicator is bromothymol blue.

10. A test strip (500) for determining the specific gravity of a urine sample urine manufactured by the method according to claims 1 to 9, the test strip determining the specific gravity of the urine sample based on the intensity of color change of the pH sensitive color indicator.

11. The test strip (500) according to claim 10, further comprising pH test pad (540).

12. A cartridge (202) for a urine optical analysis device (100), the cartridge (202) comprising a plurality of chambers (310) arranged next to one another in a right circular cylinder shape of at least 80% of a circle with a diameter comprised between 3 and 10 cm, wherein at least a chamber (310) comprises a test strip (500) according to claim 10 or 11.

13. The cartridge according to claim 12, further comprising a pH measuring strip arranged in at least a chamber.

14. A urine optical analysis device (100) comprising:
- a cartridge (202) according to claims 12 or 13,
- a station (200), configured to be positioned on a wall of a toilet bowl (102), the station (200) comprising:
+ a case (204) comprising a compartment (212) in which the cartridge (202) is at least partially received,
+ an injector, configured to inject fluid on the test strip (600),
+ an analyzer (400) to analyze a change of color of the test strip (600) due to fluid from the fluid sample.

15. The urine optical analysis (100) device according to claim 14, wherein the analyzer (400) includes a light source (402, 404) and a light sensor, configured to emit and receive light.
